# EUROPEAN PATENT APPLICATION

(11) **EP 1 205 191 A1**
(43) Date of publication of application: **15.05.2002**
(21) Application number: 00403156.3
(22) Date of filing: 13.11.2000
(51) Int. Cl.: A61K 47/48

(54) **Targeted modification of intracellular compounds**

(71) Applicant: CENTRE NATIONAL DE LA RECHERCHE SCIENTIFIQUE, 75016 Paris Cédex (FR); MASSACHUSETTS GENERAL HOSPITAL, Boston, MA 02114 (US); Molecular Sciences Institute, Berkeley, CA 94704 (US)
(72) Inventor: Colas, Pierre, 69370 Saint Didier Mont D'or (FR); Brent, Roger, Berkeley CA 94704 (US); Cohen, Barak, A., Cambridge MA 02139 (US)
(74) Representative: Almond-Martin, Carol

(57) **Abstract**

The present invention relates to a process for specifically modulating the properties of an intracellular target molecule T, and / or of a cellular component C which interacts directly or indirectly in a cell with T, said process comprising :
introducing into a cell a chimeric molecule, a so-called « targeted effector », comprising
- a recognition moiety R having the capacity to specifically interact, within the cell, with a site on an intracellular target molecule T, R interacting with T with a first affinity A₁ and
- an effector moiety, E, covalently linked to said recognition moiety R, E being a molecule, or a portion thereof, which has an initial capacity to exert an effect on at least one molecule M, and which when it is covalently linked to R, acquires the capacity to specifically exert the effect on the intracellular target molecule T,
wherein the targeted effector interacts with T with a second affinity A₂, the affinity A₁ or the affinity A₂ corresponding to a K_{d} of less than 1 x 10⁻⁸ M, and the properties of T and / or of C are specifically modulated by the effector moiety E.

## Description

The present invention relates to methods for the controlled modulation of cellular physiology using targeted modification of intracellular components. The invention als concerns molecules capable of bringing about such targeted modifications.

Specific targeting of intracellular molecules provides a powerful means of modifying cellular function and physiology. Attempts have been made to achieve this aim using molecules which have the capacity to bind a given target protein with high specificity within a cell. Such specific-binding molecules have been identified by screening large complex libraries, for example peptide aptamers (ref. 1), or arrays of synthetic compounds, for example those generated by combinatorial chemistry, or variants of binding domains of known proteins. A rational design approach can also be used, whereby knowledge of the structure of both the target and binding molecules is used to construct rational derivatives, such as variants of the PDZ domain (ref. 36). A further example is provided by intracellular antibodies or « intrabodies » comprising the variable domains of immunoglobulin heavy and light chains synthesised as a single polypeptide (ref. 41).

Binding of these intracellular « recognition » molecules to the target compound generally brings about a modification in the function of the target, for example as a result of the occlusion of an active site, the blocking or stabilization of interactions with other cellular components (for example protein-protein or protein-DNA interactions), or the fixation of the target in an active or inactive conformation.

Whilst a considerable array of different intracellular effects can be achieved using these targeting molecules, there is nevertheless a limit to the extent of the functional manipulations which can be carried out in this way. Indeed, the modulation is necessarily limited to one which can be achieved by binding of the recognition molecule to its target. Many desirable intracellular changes, such as covalent modification of an endogenous target, exposure of the target to a particular enzyme, destruction of the target or transport of the target to a given sub-cellular compartment, cannot be achieved using the existing recognition molecules alone.

A further limitation of the known systems is the binding affinity of the artificial protein recognition molecules to their target molecules. This affinity is often in the micromolar range, although can be higher for peptide aptamers. Whilst a binding affinity in this range is adequate for some cellular targets, many intracellular proteins bind their natural targets with a higher affinity. Competitive inhibition of such interactions by artificial recognition molecules requires binding affinities higher than those previously reported.

There is thus a need to extend the ability to control biological processes by intracellular targeting molecules, both in terms of the type of modification which can be achieved and the strength of the interaction with the target molecule.

The present inventors have developed a system which overcomes the limitations of the known targeting molecules.

According to the present invention, an intracellular recognition molecule which interacts with its target with an affinity of less than 1 x 10⁻⁶ M, and preferably less than 1 x 10⁻⁸ M, is covalently linked to an effector molecule, capable of exerting a desired effect. The recognition module directs the effector module to its specific intracellular target, where the combined recognition and effector modules can exert the desired effect in a specific manner and with an affinity sufficiently high to bring about the desired change to the appropriate extent.

The specific targeting molecules of the invention are thus composed of two functional entities, the first being the recognition moiety, which will be designated « R », and the second being the effector moiety which will be designated « E ». The moieties « R » and « E » are covalently linked to each other, either directly or through linker elements, and are together designated a « targeted effector ».

According to the invention, the targeted effector is introduced into a cell, where the recognition moiety confers the necessary specificity and affinity to the effector moiety, thus allowing a specific change to be brought about on the target molecule T, or a cellular component, C, which interacts with the target T, either directly or via an associated complex.

The technique of the invention is particularly advantageous in that the affinity of the interaction between the targeted effector (i.e. the covalent association of the recognition moiety and the effector moiety), and the target has been found to be directly proportional to that of the recognition moiety before fusion to the effector moiety. This is particularly surprising in view of previous reports which suggested that the fusion of intracellular recognition molecules to other molecular entities would lead to a considerable lowering of binding affinity.

It has also been found in the context of the present invention that the degree of change brought about by the combined action of the recognition moiety and the effector moiety is proportional to the affinity of the recognition moiety for the target. Thus, in accordance with the invention, not only can the nature of the intracellular change be controlled by use of an appropriate effector moiety, but the extent of the change can also be regulated by choice of a recognition moiety having a suitable binding affinity for its target.

Surprisingly, the inventors have also established that the specificity of the recognition moiety for its target is not adversely affected by the fusion to the effector moiety. Even in cases where the effector moiety has an inherent specificity different from that of the recognition moiety, it has been observed that, by virtue of its covalent link to the recognition moiety, the effector moiety acquires the capacity to exert its effect on the target molecule T. The invention thus enables the intracellular specificity of an effector molecule to be redirected.

Generally speaking, the invention relates to a process for specifically modulating the properties of a target molecule T, and / or of a component C which interacts directly or indirectly with T, said process comprising :
contacting T with a chimeric molecule, a so-called « targeted effector », comprising
   - a recognition moiety R having the capacity to specifically interact with a site on the target molecule T, R interacting with T with a first affinity A₁ and
   - an effector moiety, E, covalently linked to said recognition moiety R, E being a molecule, or a portion thereof, which has an initial capacity to exert an effect on at least one molecule M, and which when it is covalently linked to R, acquires the capacity to specifically exert the effect on the target molecule T,
wherein the targeted effector interacts with T with a second affinity A₂, the affinity A₁ or the affinity A₂ corresponding to a K_{d} of less than 1 x 10⁻⁸ M, and the properties of T and / or of C are specifically modulated by the effector moiety E.

The process of the invention is suitable for use *in vitro* and *in vivo.* For the modulation of the properties of an intracellular target T, and / or of a cellular component C which interacts directly or indirectly in a cell with T, the process of the invention comprises :
introducing into a cell a chimeric molecule, a so-called « targeted effector », comprising
   - a recognition moiety R having the capacity to specifically interact, within the cell, with a site on an intracellular target molecule T, R interacting with T with a first affinity A₁ and
   - an effector moiety, E, covalently linked to said recognition moiety R, E being a molecule, or a portion thereof, which has an initial capacity to exert an effect on at least one molecule M, and which when it is covalently linked to R, acquires the capacity to specifically exert the effect on the intracellular target molecule T,
wherein the targeted effector interacts with T with a second affinity A₂, the affinity A₁ or the affinity A₂ corresponding to a K_{d} of less than 1 x 10⁻⁸ M, and the properties of T and / or of C are specifically modulated by the effector moiety E.

The present invention thus enables the controlled modulation of the properties of an intracellular target molecule T or a molecule C interacting with T in the cell.

In the context of the invention, the following terms encompass the following meanings :
- *« modulation of the properties »* of a molecule T or C signifies the modification of one or more of the chemical, biochemical, physical and / or functional properties of T or C ;
- « *the chemical properties »* of a molecule T or C are properties arising from the chemical composition of the molecule. Modification of the chemical properties of T or C includes the formation or breakage of one or more covalent bonds or non-covalent bonds, the incorporation or loss or exchange of atoms, functional groups or residues ;
- « *the biochemical properties »* of a molecule T or C are properties arising from the chemistry of the molecule as part of a living organism, or cell or tissue, for example the capacity of T or C to combine with, or to act on, or to be acted upon, by other substances in the cell ;
- « *the physical properties »* of a molecule T or C are the properties which together define the physical or material condition of the molecule, its energy, its spatiotemporal properties, for example the sub-cellular localization of T or C in a cell, its exposure at the surface of the cell, its internalization, conformation, oligomerization state etc. ;
- *« the functional properties »* of a molecule T or C signifies any one or more of the functions of T or C, particularly as exhibited in a cell, for example at any given stage of development or in a given cellular compartment or in a given set of environmental conditions. The modificiation of the functional properties of T and / or C includes the enhancement or reduction in the efficiency of a function, or the abolition or creation of a function. An example of a function which can be modulated according to the invention is the ability of T or C to interact with cellular components such as proteins or nucleic acids, resulting in a significant change in the molecule's ability to act as an enzyme or co-factor, to regulate gene expression, to transport intracellular components, to synthesise cellular components etc.... The chemical, biochemical and physical properties of an intracellular target T or component C are usually directly related to the function of the molecule. Consequently, the modification of the chemical, biochemical and / or physical characteristics may give rise to a change in the function of that molecule..
- A binding molecule is said to « *interact »* with a target molecule when the binding molecule binds non-covalently to the target. Protein-protein and protein-DNA interactions are typical of this type of binding. The interaction occurs between the binding domain of the binding molecule and a precise site, or « epitope » on the target molecule.
- A binding molecule is said to interact « *specifically »* with a site on a target molecule when the binding molecule only has the capacity to interact with that site, i.e. the binding domain of the binding molecule cannot interact with any other site. The site (or « epitope ») in question may occur on a plurality of target molecules, in which case the binding molecule specifically recognises a range of target molecules, and can be considered to be a « pan-interacting » molecule. Alternatively the epitope may occur exclusively on one target molecule, and the binding molecule thus can only recognise that target. Occasionally, a binding molecule has the capacity to bind with high affinity to a given epitope, and also has the capacity to bind to a different epitope with lower affinity. In such a case, the interaction between the binding molecule and the target site is said to be « *specific »* when the binding affinity is at least in the micromolar range (i.e. a dissociation constant K_{d} equal to or less than 1 x 10⁻⁶ M, preferably equal to or less than 1 x 10⁻⁷ M and most preferably equal to or less than 1 x 10⁻⁸ M).

According to the invention, the recognition moiety R interacts with the target T with an affinity A₁, and the targeted effector interacts with T with an affinity of A₂.

In accordance with the present invention, binding affinities « A » are expressed as K_{d}s (M), as measured *in vitro* at equilibrium. K_{d}s can be calculated by any conventional means, such as dividing dissociation rate constants (s⁻¹) by association rate constants (M⁻¹ s⁻¹). Thus, the higher the binding affinity A, the lower the corresponding K_{d}.

It is a preferred feature of the invention that either A₁ or A₂ or both A₁ and A₂, have a K_{d} value of less than 1 x 10⁻⁸ M. A₁ may be substantially equal to A₂ or may be less than A₂. Preferably, A₁ has a K_{d} value of less than 1 x 10⁻⁸ M, most preferably less than 5 x 10⁻⁹ M, for example less than 1 x 10⁻⁹ M. According to a preferred embodiment of the invention one or both of the binding affinities A₁ and A₂ correspond to K_{d} values comprised between 1 x 10⁻⁹ M and 1 x 10⁻¹⁴ M, for example between 1 x 10⁻⁹ M and 1 x 10⁻¹² M, or between 1 x 10⁻⁹ M and 1 x 10⁻¹¹ M.

Binding affinities of recognition moieties and targeted effectors can be measured *in vitro,* using any appropriate means known in the art, for example by carrying out S.P.R. or evanescent wave experiments such as those described below in the Examples, and in reference (1). This type of method is generally used in accordance with the invention for the determination of the binding affinity A₁ i.e. the K_{d}s for a « naked » recognition moiety R, without the covalently fused effector moiety E. However, depending on the nature of the effector moiety E, the method can in some cases also be used to determine binding affinity A₂ i.e. K_{d}s for the targeted effector.

If the interaction of the recognition moiety R, or the targeted effector, with the target T gives rise to a detectable phenotype, the binding affinities A₁ and / or A₂ can also be evaluated using the extent of phenotypic interaction as an indication of the strength of the binding.

In so far as the K_{d} of the « naked » recognition moiety R correlates closely with that of the fused targeted effector, it is not indispensable to quantitatively determine both A₁ and A₂. As long as one of these K_{d} values is less than 1 x 10⁻⁸ M, the binding and modulation properties of the targeted effector will be suitable for carrying out the process of the invention.

According to a particular variant of the invention, the binding affinity of an interaction between R and T can be improved by modifying the chemical composition of the binding region of R, for example by mutagenesis using appropriate techniques such as mutagenic PCR etc, and screening the thus-obtained mutants for variants having the desired binding characteristics.

According to a first variant of this aspect of the invention, the recognition moiety R is mutated to give a mutant recognition moiety Rₘ, Rₘ having the capacity to specifically interact with a site on the intracellular target molecule T, with an affinity Aₘ, which is stronger than the affinity of the interaction between R and T (A₁). Expressed in terms of Kd, the Kd value corresponding to Aₘ is less than that corresponding to A₁.

Alternatively, according to a second variant of this aspect of the invention, the recognition moiety R may already be a mutant of a parent recognition moiety Rₚ, Rₚ specifically interacting with a site on the intracellular target molecule T, with an affinity Aₚ, which is not as strong as the affinity of the interaction between R and T (A₁). Expressed in terms of Kd, the Kd value of Aₚ is greater than that of A₁, i.e. greater than 1 x 10⁻⁸ M.

Using the technique of mutagenesis, the inventors have succeeded in preparing recognition moieties R, particularly peptide aptamers, which have binding affinities of less than 1 x 10⁻⁸ M, in particular less than or equal to about 5 x 10⁻⁹ M. This technique can be used to produce peptide aptamer recognition moieties having affinities as high as 1 x 10⁻¹² or 1 x 10⁻¹³. Enhancement of the binding affinity of the parent molecule is generally from around ten- to around twenty- or twenty-five fold after mutagenesis and sorting.

As shown in the Examples, mutagenesis of the binding domain of R may leave the specificity characteristics of R unaltered. Alternatively, mutagenesis may affect the specificity of R, either enhancing or reducing it. For example, the binding domain of a first recognition moiety R may initially interact exclusively with a first target site S₁ on the target T. Upon mutagenesis of the binding domain, the mutated recognition moiety Rₘ may acquire the capacity to bind to a second binding site S₂, present on the same target molecule T, but also present on other molecules which previously were not targets for R.

The modification of the binding behaviour of the recognition moiety when optimisation of the binding affinity is carried out, can be detected easily by specificty screens. Depending upon the objective sought, the change in specificity can either be exploited advantageously, or can be eliminated by sorting the mutants to identify those conserving the initial specificity but having enhanced affinity.

In accordance with the invention, the recognition moiety R having enhanced affinity, is covalently bound to an effector moiety, providing a targeted effector of particularly high affinity for example less than 1 x 10⁻⁹ M.

The recognition moiety R is, generally speaking, any molecule which has the capacity to interact with high specificity and affinity within a cell with a target T. Preferably, the recognition moiety R is, or comprises, a protein or a peptide, and includes protein / non-protein composites. The recognition moiety may comprise the binding region of a protein, or a modified version thereof, or the antigen-binding domain of an antibody. Alternatively, R may be an entirely synthetic molecule generated at random or through rational design.

According to a particularly preferred embodiment, the recognition moiety R comprises or consists of a randomly generated peptide, or a part thereof. By randomly generated peptide is meant a peptide having a random sequence of amino acid residues. Such peptides are encoded by random DNA sequences such as can be generated by an oligosynthesizer. A random sequence has no significant bias in the composition of the sequence. Random peptides may or may not exhibit homology with known proteins or naturally occurring protein binding domains. Such peptides can be selected from combinatorial libraries, providing a huge pool of potential recognition molecules. Preferably, the random peptides have no significant homology with naturally occurring petides.

The recognition peptides R are generally relatively small in size, for example from 5 to 150 amino acids, preferably around 40 to 140 amino acids, although may in some instances be larger or smaller. Ideally the peptide recognition moieties are easily expressed and purified in bacterial cells and in mammalian cells.

Advantageously, the recognition moiety R comprises at least one recognition region, displayed in a conformationally constrained manner in a platform, « P ». The composition of the recognition region « V » can be varied to provide the appropriate recognition characteristics, whilst the platform « P » remains constant. The recognition region will be referred to herein as the « variable region » or « V ». Recognition moieties comprising one or more variable recognition regions « V », conformationally constrained in a platform P will be designated « aptamers ». If, in such a recognition moiety, the one or more of the variable regions V comprises a peptide, the molecule will be referred to as a « peptide aptamer ».

The variable region « V » is conformationally constrained in the platform P, also known as a scaffold. This means that V has reduced flexibility i.e. can assume a limited number of conformations, because at least one of its extremities is covalently linked to the platform. Preferably, « V » is doubly constrained i.e. both extremities of the variable region are covalently linked to the platform. For example, for peptide aptamers, both the N-and the C-terminal extremities of « V » are covalently linked to the platform. Typically, the variable region is inserted in the central portion of the scaffold.

The conformational constraint of R may or may not involve the formation of disulphide bridges.

The variable region « V » is preferably a peptide having from approximately 5 to approximately 60 amino acids, preferably 10 to 40 amino acids, for example 20 amino acids or thereabouts.

The platform P (also known as a « scaffold ») can be any molecule which is capable of reducing, through covalent bonding, the number of conformations which « V » can assume. Examples of conformation-constraining scaffolds include proteins and peptides, for example thioredoxin and thioredoxin-like proteins, nucleases (e.g. RNaseA), proteases (e.g. trypsin), protease inhibitors (e.g. eglin C), antibodies or structurally-rigid fragments thereof, fluorescent proteins such as GFP or YFP, and conotoxins. A conformation-constraining peptide can be of any appropriate length, for example from 5 to 150 amino acids, preferably 5 to 40 or 5 to 60 amino acids. Even shorter stretches of amino-acid residues can provide conformational constraint. Other suitable platform molecules include carbohydrates such as sepharose, magnetic beads, fluorescent beads or gold beads. The platform P may be a linear or circular molecule, for example, closed to form a loop.

In addition to its role in conformation constraint, the platform may also be capable of conferring protection from proteolytic degradation and the like, of conferring desirable solubility characteristics etc.

Thioredoxin (TRX) and thioredoxin-like (TRX-like) proteins are particularly preferred as platforms of the present invention. Thioredoxin-like proteins are defined herein as proteins having at least 18%, preferably at least 40% and most preferably at least 75% homology with the amino acid sequence of E.coli thioredoxin over an amino acid sequence length of 80 amino acids (ref 43). Thioredoxin-like molecules also include peptides which have a three-dimensional structure substantially similar to that of human or E.coli thioredoxin, for example glutaredoxin (ref 44 ). A particularly preferred thioredoxin platform is human thioredoxin.

In TRX- or TRX-like recognition moieties, the variable region « V » is usually inserted at the active site loop of the TRX or TRX-like protein.

According to a preferred embodiment of the invention, peptide aptamers as described above are selected from combinatorial libraries by two-hybrid methods, using aptamer derivatives that also bear acidic activation domains, and using LexA derivatives of the desired target proteins as baits. Such selection ensures that the aptamers function *in vivo.* Peptide aptamers typically exhibit Kds for their target of about 1 x 10⁻⁷ to 1 x 10⁻⁸ M (ref. 1). These molecules can discriminate between closely related members of protein families (1), and even between different allelic forms of proteins (2, and Examples below). Peptide aptamers bind to a specific site on a target molecule and are therefore capable of inhibiting the interaction of a target with one interaction partner, but not all. The functional modulation of an intracellular target can therefore be carried out in an extremely precise manner.

Peptide aptamers have been shown to be capable of disrupting specific protein interactions *in vivo* (reviewed in ref. 10). For example Anti-Cdk2 aptamers competitively inhibit the interaction of Cdk2 with one of its substrates and, when expressed in human cells, delay progress through the cell cycle (3). Similarly, anti-E2F, anti-HPV E6, and anti-Ras aptamers disrupt the function of their protein targets in mammalian cells (4, 5) and anti*-Drosophila* Cdc2 and Cdc2c aptamers inhibit the function of their targets in imaginal disks (6). Finally, it has been shown that aptamers can be used as dominant genetic agents to cause a phenotype and to identify, in subsequent two hybrid assays, the proteins and interactions they target (7, 8, 9). These agents are thus well-suited for carrying out the controlled modulation of cellular physiology according to the invention.

According to the invention, the recognition moiety R, which is preferably a peptide aptamer, is covalently bound to an effector moiety, E to provide the targeted effector. The effector moiety E is any molecule, or part of a molecule, which has an initial capacity to exert an effect on at least one molecule M, thus affecting the chemical biochemical, physical or functional properties of M.

In accordance with the invention, E is said to have an « initial » capacity to exert an effect on M. In this context, an « initial » capacity signifies that E has this capacity prior to its being covalently linked to the recognition moiety R. The « initial » capacity of E to exert an effect on M may be an inherent capacity, or may be a capacity which is induced for example in response to an external stimulus such as heat or light etc. The « initial » capacity may also be a capacity which has been artificially conferred on E, for example by modification of the molecule E.

Generally speaking, E has an initial capacity to exert an efect *in cis* and / or *in trans* on M, and when it is covalently bound to R, E acquires the capacity to exert the same effect in trans on the target T. E may or may not retain its ability to act on M after fusion to R.

According to the invention, an effector moiety E, which has the initial capacity to exert an effect « *in trans »* on a molecule M is a molecule which, when it is not covalently bound to the recognition molecule R, has the ability to act on, or to be acted upon by, a molecule M which is not covalently bound to E, or to a molecule comprising E. Likewise, an effector moiety E which has the inherent capacity to exert an effect « *in cis »* on a molecule M, is a molecule which, when it is not covalently bound to the recognition molecule R, has the ability to act on, or to be acted upon by, itself or on a molecule M comprising E.

The molecule M upon which E initially has the capacity to exert an effect may be an intracellular or extracellular molecule. It may be the same or different from the molecule T which is the target of the recognition moiety R.

The inventors have established that in some cases, effector moieties which normally exert an effect *in cis,* i.e. on a molecule which is covalently bound to the effector, acquires the ability to exert that same effect *in trans* on the recognition moiety's target T. The *cis* effect is thus converted to one which can be exerted without covalent bond to the target molecule.

In the targeted effector, E is covalently linked to the recognition moiety R, for example either directly to the amino or carboxy terminal of R, or alternatively via linking sequences which may themselves also provide a particular function. E may also be inserted within R, provided that the function of the variable region V is not disrupted by the insertion. E may thus be conformationally constrained at one or both extremities.

E is preferably, but not necessarily, a protein or a peptide, or comprises a protein or a peptide. For example, the effector moiety E may comprise or consist of an enzyme, a catalytic domain thereof, a co-factor, an addressing signal, a transcription regulatory protein, a tracer protein, a molecule having therapeutic or diagnostic properties, a second recognition moiety, a second targeted effector, a radionuclide, a chemical modifier, an enzyme which can act upon itself etc. When E is or comprises a peptide, its size can vary from two to three amino acids to upto several hundred amino acid residues or more. Typical sizes range from about twenty to 200 amino acids, for example, 30 to 100 amino acids.

Examples of initially trans-acting effector moieties are enzymes, transcription factors or components thereof, substrates for enzymes, co-factors etc. Examples of initially cis-acting effector moieties are addressing signals or intracellular transporters, labels, markers etc.

The inventors have established that upon linking the effector moiety E to the recognition moiety R, E acquires the specificity of R, that is, it acquires the capacity to act, in a cell, upon the same range of intracellular targets as R. This effect is particularly surprising when E is a molecule which has an intrinsic specificity different from that of R. For example, the inventors have observed that an enzyme which normally is incapable of interacting with a given intracellular target T acquires the ability to exert its usual enzymatic activity on T when it is joined to the recognition moiety. The specificity of the effector moiety can thus be redirected in accordance with the invention. In addition to acquiring the specificity of R, the covalently linked E may in some cases also conserve the ability to exert an effect on its initial partner M. Whether or not E retains the ability to act on M depends upon the nature of E, M, and R and the nature of the cell. Moreover, if the effector moiety E is a molecule which has an endogenous counterpart in the cell, the specificity of the endogenous counterpart is not affected by the presence in the cell of the targeted effector carrying E with a redirected specificity.

The targeted effector of the invention may carry one or more effector moieties, for example two or three. If more than one effector moiety is present, they can be functionally co-ordinated or functionally independent, provided that the functions are compatible with each other. An example of functionally co-ordinated effector moieties is provided by a first effector moiety E₁ which is responsible for directing the target T to a particular sub-cellular compartment, and a second effector moiety E₂ which can act on the target T, or a different molecule, only once it is in that cellular compartment. An example of this type of system is where E₁ is a nuclear localising signal, E₂ is a component of a modular transcription factor, and the target T is a cytoplasmic protein which comprises a second component of the modular transcription factor. The targeted effector interacts with the cytoplasmic target T, thereby reconstituting the modular transcription factor and simultaneously carrying the cytoplasmic target molecules into the nucleus. The transcription factor can then bind to its DNA target and regulate transcription.

If the targeted effector carries more than one effector moiety, they may all be initially *trans-acting,* or may all be initially *cis-acting,* or some may be *trans-* and some may be *cis-acting.* Furthermore, some or all of the effector moieties may be initially both *cis-* and *trans-acting.*

The effector moiety E may be, or may include, a molecule which induces the destruction or inactivation of the targeted effector once the desired intracellular modulation has been carried out. Such an effector moiety may be used to prevent long-lived recognition molecules from causing undesirable interactions within the cell. An example of an effector moiety which is capable of modifying the target molecule and of destroying the recognition moiety is ubiquitin ligase.

According to a further variant of the invention, the effector moiety is a molecule other than a transcription activator.

Particularly advantageous embodiments of the invention are the « modifier » and « transporter » embodiments. These variants will be described below.

The modifier embodiment of the invention involves the use of targeted effectors to bring about a change, particularly the creation of a covalent bond, in an intracellular target. It preferably involves the use of effector moieties which cannot normally act on that target, but which acquire the capacity to do so by virtue of their link to the recognition moiety. In other words, this variant preferably comprises the specific modification of an intracellular target by effector moieties of redirected specificity.

More specifically, accoding to the modifier embodiment, the effector moiety E has the capacity to exert an effect on the target T in a cell when it is covalently bound to the recognition moiety, but cannot exert this effect on T or C in the same cell in the same temporal and spatial environment when it is not covalently bound to the recognition moiety. Preferably, according to the « modifier » variant of the invention, E is initially *trans-acting.*

According to this embodiment, the inability of the effector moiety E to exert an effect on T or C in the cell when it is not covalently bound to the recognition moiety is generally due to the inherent specificity of E or T, for example in the case of an enzyme, whose inherent specificity prevents the target protein T from acting as substrate for E when it is not covalently linked to the recognition moiety.

Alternatively, the effector moiety E may be initially incapable of exerting an effect on T or C in the cell on account of a temporal or spatial specificity of T and / or E which prevents E and T from associating with each other when E is not covalently linked to the recognition moiety. Likewise, if E and T or C are not co-expressed in the cell, E will not be able to exert an effect on T and / or C.

The covalent association of the effector moiety E and the recognition moiety R permits the initial inability of E to act on T, to be overcome.

According to the transporter embodiment of the invention, the effector moiety E has the initial capacity to transport an intracellular molecule M *in cis* to a predetermined sub-cellular compartment. On covalently joining E to R, E acquires the capacity to direct the target molecule T to the said sub-cellular compartment. According to this embodiment, E is therefore a transporter molecule or an addressing signal, such as NLS, secretory signal etc. The covalent association of the effector moiety and the recognition moiety enables E to exert this effect *in trans* on the recognition moiety's target T.

Preferably the sub-cellular compartment to which the target is transported by the effector moiety is one to which the target would not normally be addressed or to which it would only be addressed in specific conditions, or to a very limited extent. The transport is thereby « forced » by the targeted effector.

Concerning the intracellular target molecule T, this molecule can be any intracellular molecule. It may be endogenous to the cell or may be heterologous to the cell, for example a recombinant protein.

T may be a protein, a peptide, a nucleic acid, a carbohydrate, a phosphorylated molecule, a lipid, or a combination of any of these, for example a protein / DNA complex. The target T may be a molecule which is present only under certain conditions in the cell, for example a protein which is expressed with spatial or temporal specificity. T may be present in any or all cellular compartments, for example cytoplasmic or nuclear or uniformly distributed throughout the cell.

In some instances, the target protein T may be a protein comprising at least two functionally distinct protein domains, Tₐ and T_{b}., for example a synthetic fusion protein produced artificially in the cell, or a naturally occurring endogenous protein having distinct functions mapping to distict domains. In such a case, it is possible that the recognition moiety R of the invention specifically binds to a site on the first functional domain Tₐ and the effector moiety E exerts a structural or functional modulatory effect on the second functional domain T_{b}. This is particularly advantageous in cases where conservation of one of the functions of the target T is desirable, whilst modifying others.

The intracellular molecule upon which the targeted effector exerts its effect may in some cases not be directly the target molecule T, but a further intracellular component C with which T interacts, directly or indirectly. C may be any intracellular component, for example a protein, nucleic acid, lipid, phosphate, carbohydrate etc.

The interaction of the targeted effector with the target T or the intracellular component C gives rise to any one or more of a number of different chemical, biochemical, physical and / or functional changes. Examples of such modifications include :
- chemical changes such as the formation or breakage of one or more covalent bonds or non-covalent bonds in T or in C,
- physical changes such as the modification of the conformation of T or C, the transport of T or C to a sub-cellular compartment, or to the cell membrane on the inside or outside of the cell, labelling of T or C etc;
- functional changes such as the enhancement or reduction in the efficiency of one or more functions of T or C, or the abolition or creation of a function ;

The modulation of the chemical, physical, biochemical or functional properties of the target T or the cellular component C may give rise to a phenotypic change in the cell or organism. This phenotypic change may or may not be selectable or detectable.

The result of the modulation brought about by the targeted effector may include one or more of the following effects :
- homo- or heterodimerisation of T or C ;
- inhibition of homo- or heterodimerisation of T or C ;
- destruction of T or C ;
- stabilisation of T or C ;
- labeling of T or C ;
- ability of T or C to interact with a new partner ;
- localisation of T or C to a subcellular compartment to which T or C are not otherwise transported in the cell ;
- localisation of T or C to a subcellular compartment to which T or C are otherwise transported to a lesser degree in the cell ;
- exposure of T or C at the cell membrane ;
- internalisation of T or C ;
- provoking the association of T or C with natural intracellular partners ;
- prevention of the association of T or C with natural intracellular partners ;
- cleavage of T or C ;
- secretion of T or C.

According to a preferred embodiment of the invention, the recognition moiety R, in the absence of the effector moiety E, has no effect on the chemical, biochemical physical or functional properties of the target T i.e. R is neutral with respect to the the target protein T. In such cases, the modulation brought about by the targeted effector is entirely due to the effector moiety E. This type of interaction is possible, for example with peptide aptamers, because they interact with specific, clearly delimited sites or epitopes on a target. It is therefore possible to select aptamers which specifically interact with a desired target molecule T, but which do not happen to affect a particular function of T. As described above, if the aptamer is subject to mutagenesis of the variable region to optimise binding affinity, a slight modification of the selectivity of the aptamer can be provoked. Such controlled modification of the specificity may be used to create high affinity aptamers which are neutral with respect to the function of the target T.

It is also possible, according to the invention, that the interaction of the recognition moiety R with the target T give rise to the modification of the function of T. In such a case, the net result of the effector moiety on the target T is the combined action of the recognition moiety and that of the effector moiety.

According to a further variant, the effector moiety E does not affect the function of the target T or of the component C. In such a case, E may for example simply provide a label for the target.

The process of the present invention is carried out in a cell. The cell may be prokaryotic or eukaryotic. Eukaryotic cells are particularly preferred, for example, mammalian cells, including human and non-human cells, insect cells, plant cells, fish cells, avian cells, yeast cells etc. The cells may be cells in culture *in vitro,* or may be in whole organisms *in vivo,* or may be taken from a whole organism and treated *ex vivo.*

The targeted effector may be introduced into the cell in a number of different ways. For example, it can be introduced into the cell by expression of a DNA sequence encoding the targeted effector in the form of a fusion protein composed of the effector moiety and the recognition moiety. This method is generally applied when genetic manipulation of the cell or the organism is possible, and when the targeted effector is a protein. In such cases the targeted effector is introduced into the cell in a suitable vector, comprising all the necessary control sequences for expression.

Alternatively, the targeted effector is introduced into the cell in purified form using a cell permeable agent, such as protein transduction domains (PTDs), for example penetratin. This method is particularly advantageous for therapy when genetic modification of the individual is undesirable. A further alternative is to microinject the targeted effector into the cell.

The invention also relates to a method of producing the high affinity intracellular targeting molecules.

In particular, this aspect of the invention concerns a process for the production of a targeted effector having the capacity to specifically modulate the properties of an intracellular target molecule T, and / or a cellular component C which interacts directly or indirectly in a cell with T, said process comprising the steps :
a) production of a random pool of peptides, so-called recognition moieties R,
b) screening of the random pool produced in (a) against T in a cell, in conditions suitable to allow identification of recognition moieties R capable of interacting with T,
c) optionally contacting the moieties selected in (b) with proteins other than T to determine the specificity range of each of said moieties, and to identify moietes having a desired specificity range,
d) covalent linkage of the recognition moieties R to an effector moiety E,
   E being a molecule which initially has the capacity to exert a predetermined effect on at least one intracellular component M,
   step (d) being carried out either prior to step (b), or after step (e),
e) verification of the affinity A₁ with which the recognition moiety R interacts with T, or of the affinity A₂ with which the targeted effector, interacts with T,
f) if both of A₁ and A₂ correspond to K_{d} values greater than 1 x 10⁻⁸ M, alteration of the binding region of the effector moiety to adjust the binding affinity of the interaction between T and the selected moiety so that the K_{d} becomes less than 1 x 10⁻⁸ M.

Step (b) in the above process may comprise the screening of the pool of random peptides in a cell against an unknown or known endogenous target molecule T, wherein interaction with T gives rise to a detectable or selectable phenotypic change. If approriate, the identity of T may then be determined by screening selected recognition moieties against molecules known to have the capacity to give rise to said phenotypic change, and deducing the identity of T. This method of producing and characterising targeted effectors can be employed when the nature of the effector moiety prevents the use of classic two-hybrid interaction trap system. Alternatively, the identity of T may be determined by using R or the targeted effector R-E as a « bait » in a two-hybrid interaction assay, and fishing potential « T's » from a library.

Alternatively, step (b) may comprise the screening of the pool of random peptides in a cell against a known artifical target molecule T, interaction with T giving rise to a detectable or selectable change. An example of this type of screening is the two-hybrid assay and its derivatives. According to this method, step (b) involves the detection of reporter gene transcription, artificial candidate target proteins being co-expressed in the cell with the recognition moieties.

The invention also relates to the targeted effectors themselves. More particularly, this aspect of the invention relates to a chimeric molecule comprising
- a recognition moiety R, having the capacity to specifically interact, within a cell, with a site on an intracellular target molecule T, the interaction with T occurring with an affinity A₁, and
- an effector moiety, E, covalently linked to R, E being a molecule which has an initial capacity to exert an effect on at least one molecule M, and which when it is covalently linked to R, acquires the capacity to specifically exert the effect on the intracellular target molecule T,
wherein the targeted effector interacts with T with an affinity A₂, the affinity A₁ or the affinity A₂ corresponding to a K_{d} value of less than 1 x 10⁻⁸ M.

Preferably, the binding affinity A₁ is less than or equal to 1 X 10⁻⁹ M, for example comprised between 1 X 10⁻⁹ and 1 X 10⁻¹³ M, for example less than 1 X 10⁻¹⁰ or less than 1 X 10⁻¹¹ or less than 1 X 10⁻¹² M.

The invention also relates to the high affinity « naked » recognition moiety « R ». This aspect of the invention concerns more specifically an intracellular recognition molecule R, composed of a conformationally constrained recognition domain, displayed in a platform,
said recognition molecule having the capacity to specifically interact, within a cell, with a site on a predetermined intracellular target molecule T, the interaction with T occurring with an affinity corresponding to a K_{d} value of less than or equal to 5 x 10⁻⁹ M.

The naked recognition moiety R preferably interacts with T with an affinity corresponding to a K_{d} value comprised between 1 x 10⁻⁹ M and 1 x 10⁻¹³ M, for example between 1 x 10⁻⁹ M and 1 x 10⁻¹² M. The characteristics of the naked recognition moiety are described in detail above for « R ».

The invention also relates to a pharmaceutical composition comprising the targeted effector of the invetion, or a nucleic acid encoding such a targeted effector, in association with a pharmaceutically acceptable excipient.

The chimeric targeted effectors of the invention and the nucleic acids encoding the targeted effectors can be used in therapy. For example, the targeted effectors or the nucleic acids can be used in methods of treatment, or in the preparation of medicaments for treating
- microbial infections, including viral infections, and fungal infections ;
- immunological disorders ;
- neurological disorders ;
- metabolic disorders ;
- psychiatric disorders ;
- myopathies ;
- genetic disorders ;
- cancer ;
- cardiovascular disorders ;
- dental disorders.

Preferred embodiments of the invention, which will be described in detail in the Examples below, are peptide aptamer derivatives which covalently modify and change the localization of target proteins *in vivo.* In accordance with the invention, the peptide aptamers were made by first generating a higher-affinity mutant anti-Cdk2 aptamer, mutagenizing the variable region of an existing aptamer and screening for tighter binding mutants using relatively insensitive two-hybrid reporter genes. This variant had a significant increase in affinity and exhibited a Kd between 2 and 5nM. Use of still-less sensitive reporter genes (28), and/or LexA mutants with a decreased affinity for their operators (29) and/or substitution of weaker activation domains on the aptamer library, should allow the identification of mutant aptamers with sub-nanomolar affinity in one step.

To construct modifiers, proteins that covalently alter target proteins *in vivo,* ubiquitination i.e. the coupling of ubiquitin molecules to lysine residues on proteins, was exploited. These modifiers ubiquitinated the LexA-Cdk2 target. Moreover, the results suggest that the modifiers destroyed themselves, by self-ubiquitination on lysine residues exposed at the surface of the thioredoxin platform. This observation is consistent with the fact that the native Rsp5 protein ubiquitinates itself *in vitro,* probably on lysine residues lying in the amino-terminal part of the protein, outside of its *hect* domain (23,30).

The invention provides the first *in vivo* demonstration of targeted protein modifications by enzymes of redirected specificity. However, these aptamer-*hect* fusions did not destroy their Cdk2 targets, even those that contained extra lysine residues. It is possible that, for Cdk2, ubiquitination mediated by a more active effector domain would result in destabilization. Consistent with this idea, Gosink et al. redirected the specificity of two plant E2 ubiquitin-conjugating enzymes, Ubc1 and Ubc4, *in vitro* by fusing different protein-binding peptides, including the Ig binding domains of *Staphylococcus aureus* A protein, to Ubc carboxy-termini (31). *In vitro,* these authors observed ubiquitination of the cognate substrates, and a partial degradation of the targeted IgG. However, the most likely explanation for the stability of ubiquitinated LexA-Cdk2 fusions is that Cdk2 is simply refractory to ubiquitin-mediated proteolysis. In fact, no Cdk is known to undergo ubiquitin-dependant proteolysis, although some of its binding partners are degraded by this means (32). A number of other proteins are also ubiquitinated without being degraded, including H2A (24), cyclins in certain cell cycle phases (33) and some membrane receptors whose ubiquitination signals endocytosis without involving the proteasome (34).

Peptide aptamers fused to a nuclear localization sequence were also used as "transporters". Anti-Cdk2 and anti-Ste5 aptamers that carried nuclear localization signals caused their targets to accumulate in the nucleus. The results suggest that it will be possible to build transporters with other protein moieties that readdress their protein targets to other subcellular compartments : the endoplasmic reticulum (36), the mitochondrial membrane (37) or the plasma membrane (38). The aptamers in these transporters all blocked the activity of their target proteins, but, as has been noted in the case of the NLS containing anti-Ste5 aptamers, inactivation may in fact be caused by enforced nuclear localization, rather than by blocking interaction with partners (7). Because transporting aptamers allow mislocalization of targeted proteins that are expressed under the control of their own promoters, the perturbations that transporters induce in cell function should be less severe than those caused by overexpression of target proteins fused to addressing sequences.

The construction of new proteins from different functional modules has been reported for many types of proteins (28,39), and the success of these methods can be taken to support the current picture in which exons are shuffled among proteins during evolution (40). Peptide aptamers represent useful general purpose recognition moieties in chimeric proteins with other effector domains with other functions. The ability to control the spatial and temporal expression of such "modifiers" and "transporters" in cells and whole organisms should facilitate a finer control of protein modification, inactivation, and localization. Furthermore, the ability to select aptamers that distinguish among allelic variants of proteins should allow selective modification of the activities of individual alleles. The generation and use of new peptide aptamer derivatives should facilitate high-resolution study of regulatory pathways, and could be useful in new therapeutic strategies

Different aspects of the invention are illustrated in the figures :

### Figure Legends

### Table 1. GFP interaction phenotypes and Kds.

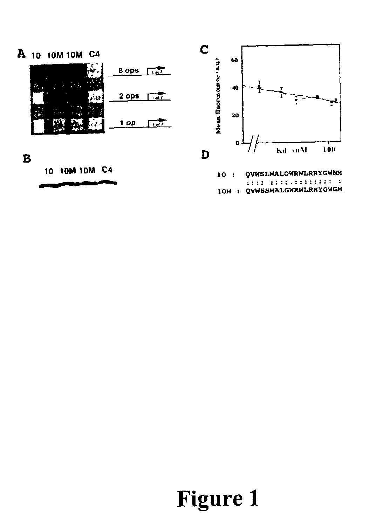

Average green fluorescence of yeast above background caused by interactions between LexA-Cdk2 and aptamers 2,3,5,8,10 and 11 in 4 independent experiments. Previously measured Kds (1) were plotted against measured fluorescence (Fig.1*c*). The plot fit the following equation Kd = 10 exp-(fluo + 123.7 / 21.9). We calculated a 2nM Kd for aptamer 10M by interpolation using this equation.
Fig. 1. A mutant aptamer with enhanced affinity for its target. *(a)* Interaction mating assay between LexA-Cdk2 and aptamer 10, two strains carrying aptamer 10M, and a non-interacting aptamer C4, using three different sensitivity LexAop-lacZ reporters. (*b*) Western blot assay using an anti-TrxA antibody. Diploid exconjugates were grown in galactose containing medium, and proteins were extracted and subjected to a Western blot analysis with anti-TrxA antibody. (*c*) Strength of interaction phenotypes as determined by fluorescence from a LexAop-GFP reporter plotted against Kds measured in evanescent wave experiments. Fluorescence values are in arbitrary units (a.u.). (*d*) Sequence of the variable regions of aptamers 10 and 10M.

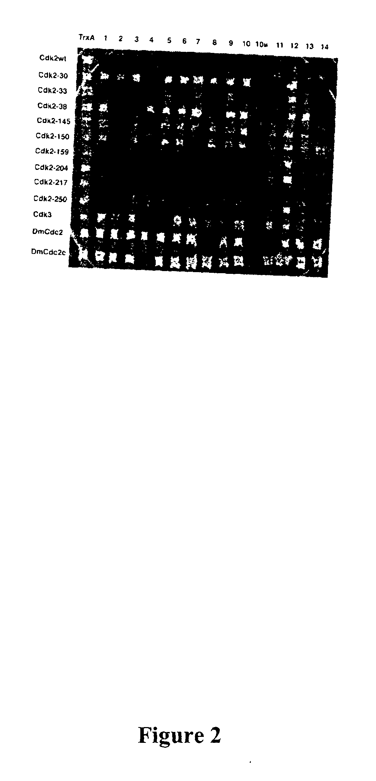

Fig. 2. Mapping sites on Cdk2 bound by the original aptamers and of aptamer 10M. The Cdk2 mutant bait proteins, described elsewhere (3), Cdk3, and *Drosophila* Cdc2 and Cdc2c (16) were collected. In this experiment, TrxA, the 14 different aptamers originally selected (1), and aptamer 10M were expressed as preys. Yeast was mated to generate an interaction matrix (16).

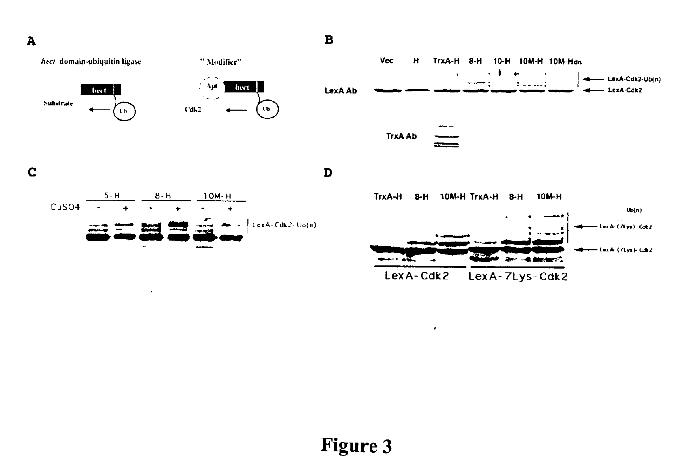

Fig. 3. Targeted ubiquitination of LexA-Cdk2 by aptamer-*hect* fusions. (*a*) Design of a "modifier", inspired by the structure of *hect* domain containing ubiquitin ligases. *(b)* Western blot analysis of LexA-Cdk2 (upper panel) and TrxA-*hect* or aptamer-hect fusions (lower panel) using anti-LexA and anti-TrxA antibodies respectively. (*c*) Western analysis of LexA-Cdk2 when aptamer-hect fusions are expressed by growth overnight in a medium that does or does not contain CuSO4 and that does or does not express Myc-tagged ubiquitin. (*d*) Western blot analysis of LexA-Cdk2 and LexA-7Lys-Cdk2 when TrxA-, 8- and 10M-*hect* fusions are expressed, using the anti-LexA antibody.

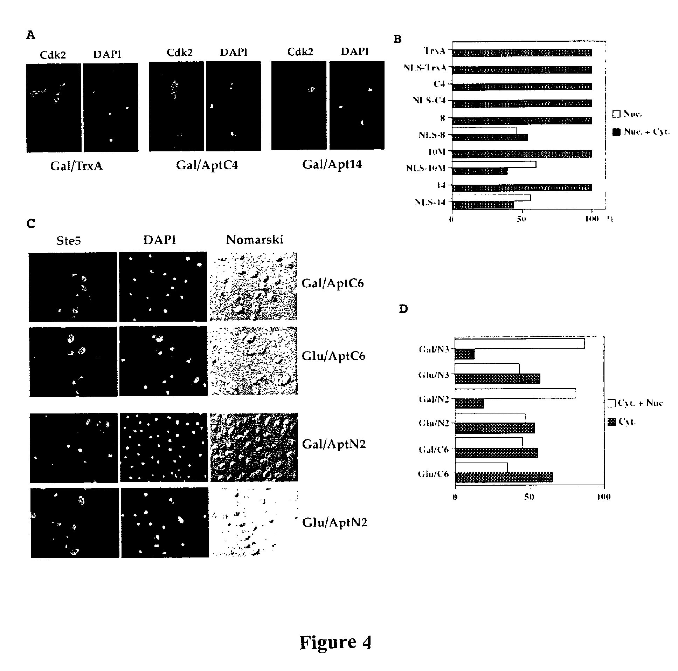

Fig. 4. Nuclear translocation of LexA-Cdk2 and LexA-Ste5 by interacting NLS-aptamer derivatives. (*a*) Yeast photomicrographs. Left panels: Indirect immunofluorescence of LexA-Cdk2 protein using anti-LexA antibody. Right panels: DNA staining with DAPI. Gal/TrxA: TrxA is expressed. Gal/AptC4: control aptamer C4 is expressed. Gal/Apt14: anti-Cdk2 aptamer 14 is expressed. (*b*) Percentage of yeast that displayed clear nuclear immunofluorescence, in presence of aptamers addressed to the nucleus or not. Dark bars: nuclear and cytoplasmic staining. White bars: nuclear staining. At least 50 cells were observed for each assay. (c) Yeast photomicrographs. Left panels: Indirect immunofluorescence of LexA-Ste5 fusion protein using anti-LexA antibody. Center panels: DNA staining with DAPI. Right panels; yeast observed with Nomarski optics. Gal/AptC6: non-NLS aptamer C6 is expressed. Glu/C6: non-NLS aptamer C6 is not expressed. Gal/AptN2: NLS-aptamer N2 is expressed. Glu/N1: NLS-aptamer N2 is not expressed. (d) Percentage of yeast that displayed cytoplasmic + nuclear, or purely cytoplasmic staining, in presence of various aptamers addressed to the nucleus or not. Dark bars: cytoplasmic staining. White bars: cytoplasmic and nuclear staining. At least 100 cells were observed for each assay.

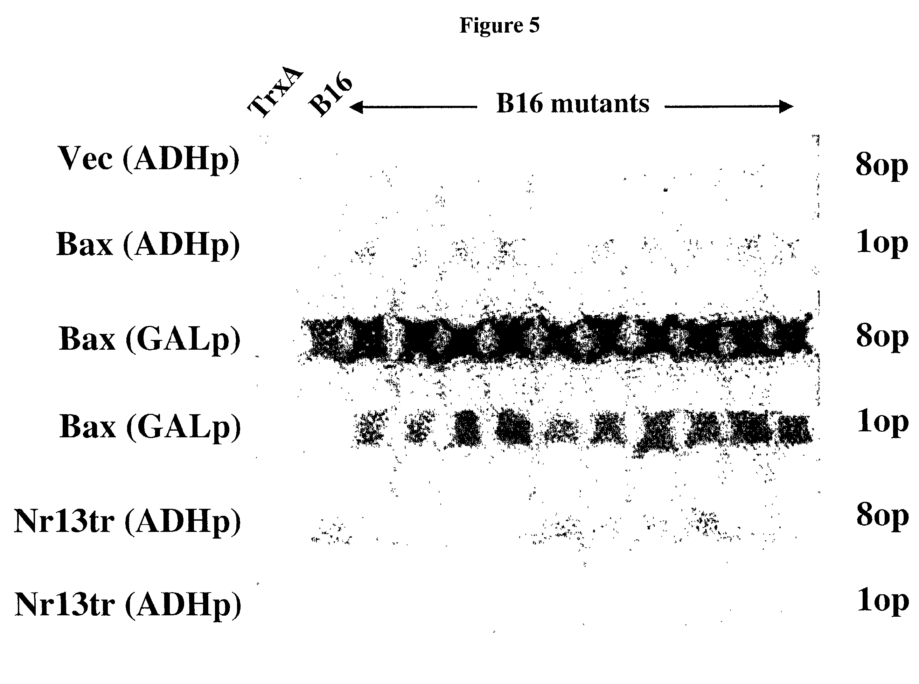

Figure 5 : Affinity of aptamers evolved in vitro. This matrix shows the improved affinity of the 10 mutants as compared to the parental aptamer (B16). On the left, the bait proteins and the promoters used to express them are indicated. On the right, the lacZ reporter genes are indicated. Considering the respective strengths of the ADH and GAL promoters on galactose medium, the sensitivity of the 2H assay can be ranked as follows, from the least to the most sensitive configuration :
   ADHp/1op
   GALp/1op
   GALp/8op
   This matrix clearly shows that all mutants bind Bax more tightly than B16. It also shows that while some mutants may bind Nr13 more tightly, others no longer bind Nr13, at least at a detectable level.

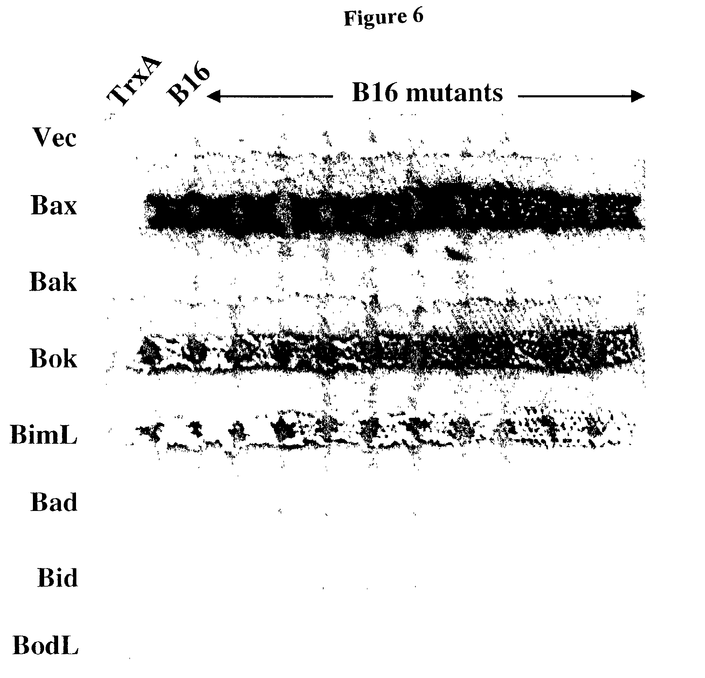

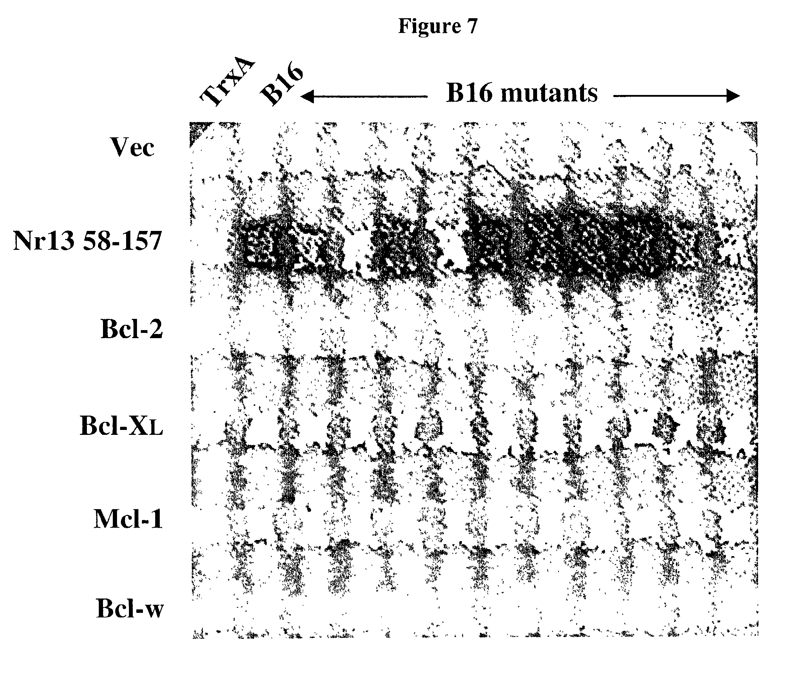

Figures 6 and 7 : Specificity of aptamers evolved in vitro. Figure 7 confirms the observation made in Figure 5. None of the mutant aptamers bind anti-apoptotic proteins (Bcl-2, Bcl-X1 etc...), other than Bax, at detectable levels, like the parental aptamer. Figure 6 shows pro-apoptotic proteins (Bax, Bak, Bok, etc...) . It can be seen that most, if not all mutants do not bind any of these proteins, like the parental aptamer. Two mutants may have acquired the ability to bind to Bok, although this cannot be determined unequivocally sinc Bok expressed as a bait protein activates transcription of the reporter gene per se.

### Examples

In the following examples, derivative peptide aptamers are described that covalently modify or change the subcellular localization of their target proteins. First, selection of a peptide aptamer with an improved affinity for its target is described. This improved aptamer and others are used to construct two types of chimeric proteins: "modifiers", which ubiquitinate their target proteins, and "transporters", which change the subcellular localization of their targets.

The materials and methods used in the following examples are described in detail in the section « Materials and Methods ».

### Results

### Example 1 : Generation and identification of a higher affinity anti-Cdk2 aptamer

An existing anti-Cdk2 aptamer was mutagenized to select one with higher affinity. The starting molecule, aptamer 10, has a measured Kd of 1.05 X 10⁻⁷M (1). Random PCR mutagenesis was performed on the aptamer 10 V region, and the PCR products were reintroduced into the library vector, pJM-1. This vector directs the expression of aptamers fused to the SV40 nuclear localization sequence, the B112 activation domain, and the HA epitope tag under the yeast GAL1 promoter (1). A pool of 15,000 mutants was created. To obtain a measure of the efficiency of the PCR mutagenesis, the V regions of two clones from this pool were sequenced ; sequencing revealed that these carried 3 and 4 mutations that resulted in 1 and 3 amino acid changes respectively. To identify tighter-binding variants from this pool, advantage was taken of the existence of different LexA operator reporter genes with different sensitivities. A strain that expressed a LexA-Cdk2 bait and that carried pRB1840, a relatively insensitive lacZ reporter (13) with a single synthetic LexA operator was used initially. This reporter was not activated by aptamer 10 (Fig. 1*a*). The pool of PCR mutagenized plasmids was introduced into this strain, and transformants that gave rise to blue colonies were identified. The V regions of the 7 plasmids thus identified carried identical nucleotide changes, and thus were presumably not independent. The nucleotide changes resulted in two amino acid changes: Leu to Ser at V region position 5, and Asn to Gly at position 19 (Fig. 1*d*). This mutant did not interact with Max and Rb, proteins unrelated to the Cdk family (not shown). The mutant aptamer was termed « 10M ».

The affinity of 10M and 10 for Cdk2 was compared by interaction mating. The reporters, pSH18-34, pJK103, and pRB1840, contain respectively 8 high affinity operators, 2 high affinity operators, and 1 lower affinity LexA operator (Fig. 1*a*). While, as judged by blue color from the pSH18-34 reporter, aptamer 10M had only a slightly greater affinity than aptamer 10, blue color from pJK103 and pRB1840 clearly indicated that 10M bound LexA-Cdk2 more strongly. To verify that the apparent higher affinity was not due to increased expression and/or stability of aptamer 10M, Western blotting experiments were performed and showed that the steady-state levels of aptamer 10, 10M, and a control aptamer were identical (Fig. 1*b*).

The gain in affinity was measured by two different means. First, a LexAop-GFP reporter gene was used to quantify interactions between anti-Cdk2 aptamers and LexA-Cdk2. Mean fluorescence obtained from each interaction was plotted against Kds measured in evanescent wave experiments (1). This plot followed a logarithmic equation (Table 1, and Fig. 1c). This equation was used to calculate the Kd of the interaction between LexA-Cdk2 and aptamer 10M from the fluorescence it conferred (Table 1). Evanescent wave experiments were also performed with purified aptamer 10M and His6-Cdk2 (12). The measured Kd was 5nM (not shown), quite close to the 2nM Kd calculated from the GFP data.

**Table 1**

| **Aptamer** | **Kd (nM)** | **Mean Fluorescence (arbitrary units)** | **Standard deviation** |
|---|---|---|---|
| 8 | 38 | 40.8 | 4.3 |
| 5 | 52 | 36.8 | 3.7 |
| 2 | 64 | 30.9 | 3.0 |
| 11 | 87 | 33.0 | 1.4 |
| 10 | 105 | 29.0 | 2.8 |
| 3 | 112 | 30.3 | 1.7 |
| *10M* | *2* | *67.5* | *3.8* |

To evaluate the contributions of the V region changes to increased affinity, we mutated both residues individually to wild type and analyzed the single mutants by interaction mating (data not shown). These experiments showed that the mutation of V region residue 5 (Leu to Ser) contributed to the gain of affinity, but that the mutation residue 19 (Asn to Gly) did not.

In order to determine whether the gain in affinity in 10M was caused by changes in its contact(s) with Cdk2, binding of all existing anti-Cdk2 aptamers, including 10M, to a collection of Cdk2 mutants, related members of this protein family, and control proteins (not shown) was examined. Figure 2 shows that, as previously observed, some of these aptamers recognize different epitopes conserved among Cdk proteins (1). However, by contrast with the aptamers isolated in previous work, aptamer 10M showed distinct crossreactivity to other Cdk proteins. This interaction with Cdk family members is consistent with three ideas. First, the Leu to Ser change might create direct contact(s) between the variable region and residue(s) conserved among the Cdk proteins tested. Second, the change might indirectly create contacts by changing the conformation of the variable region. Third, even though Leu is more dihedrally constrained than Ser, a change to Ser might create new internal contacts within the variable region and thus reduce its conformational entropy.

### Example 2 : Targeted intracellular protein modifiers

Aptamer 10M and others were used to make protein derivatives that ubiquitinated target proteins *in vivo.* The design was based on the structural organization of the *hect* domain-containing ubiquitin ligases, which conjugate ubiquitin received from a E2 enzyme and transfer it to a protein substrate (22). The amino-terminal substrate-recognizing region of these enzymes varies greatly, whereas the carboxy-terminal region, the *hect* domain, which carries the catalytic activity, is conserved between family members and throughout evolution (23). This modular structure suggested that we could fuse a *hect* domain to peptide aptamers and create ubiquitin ligases with engineered specificities (Fig.3*a*).

Accordingly, a hect domain native to the yeast protein Rsp5 was used to construct various aptamer-*hect* fusions. These were exprssed in yeast together with their putative LexA-Cdk2 targets.

The specificity of the aptamer-hect fusions, as evaluated indirectly by the capacity to ubiquitinate, was seen to reflect that observed in the interaction matrix generated in yeast assays using the corresponding aptamer without hect. The aptamer-hect fusions were able to discriminate between closely related target molecules such as Cdk2 and Cdk3.

The fates of these fusion proteins was examined. Figure 3*b* shows that while the control TrxA-*hect* fusion that lacked a variable region was detectable by Western analysis, none of the anti-Cdk2 aptamer-*hect* fusions could be detected. This result indicates that attachment of a TrxA aptamer to a *hect* domain destabilizes the aptamer. However, expression of anti-Cdk2 aptamer-*hect* fusions resulted in the appearance of a ladder of higher molecular weight forms of LexA-Cdk2, suggesting that these chimeric proteins, even expressed at low levels, still caused ubiquitination of the target. The ladder of higher molecular weight LexA-Cdk2 forms was most apparent when the 10M-*hect* fusion was expressed, suggesting that the affinity of the modifier for the target affected the degree of target modification. Modification did not result in destruction: as determined from Western (Fig.3*b*) and pulse chase experiments (not shown), expression of these modifiers had no effect on LexA-Cdk2 stability or half life.

To confirm that this ladder of higher molecular weight species corresponded to LexA-Cdk2-ubiquitin conjugates, two different experiments were performed. First, a yeast strain was used that conditionally expressed Myc-tagged ubiquitin, under the control of a copper-inducible promoter (15), and repeated the above experiments. This ubiquitin derivative is larger than native ubiquitin, and conjugates that contain it have higher molecular weights (15). On addition of CuSO4 to the culture medium, the bands on the ladder of higher molecular weights shifted higher than those in the uninduced culture (Fig.3*c*). Second, a loss of function anti-Cdk2 aptamer-*hect* fusion was generated by changing the cysteine residue that forms the thioester bond with ubiquitin to alanine (23). In cells that expressed this mutant fusion, we did not observe the LexA-Cdk2 ladder, but now did observe, for the first time, the enzymatically-dead aptamer-hect derivative (Fig. 3*b*). This fact suggests that active aptamer-*hect* derivatives may be sensitive to proteolysis after self-ubiquitination. This idea is supported by two lines of evidence. First, ubiquitination occurs on lysines (24), and, by contrast with aptamer 5-*hect*, 8*-hect, 10-hect,* and 10M*-hect* fusions, aptamer 2-*hect* and 11-*hect* fusions, which contain lysines in their V regions, did not ubiquitinate LexA-Cdk2 (not shown), even though they bind Cdk2 as tightly as the others (Fig. 2). This fact is consistent with the idea that ubiquitination of their V regions blocks their binding. Second, variant aptamer-*hect* fusions in which we changed different combinations of the 5 solvent-exposed lysines (K19, 37, 53, 70, 83) (25) on TrxA to arginines resulted in proteins detectable by anti-TrxA antiserum (data not shown). These observations suggest that the aptamer-*hect* fusions are vulnerable to ubiquitination by their own *hect* moieties and subsequently proteolyzed, but are able to ubiquitinate their targets even though expressed at very low steady states.

Finally, it was tested whether LexA-Cdk2 could be sensitized to ubiquitin-dependent proteolysis by introducing into it extra lysines as ubiquitin acceptors. To this end, a LexA-Cdk2 derivative that carried 7 lysines between the LexA and the Cdk2 moieties was expressed in yeast. This LexA-Lys7-Cdk2 target was co-expressed together with TrxA*-hect*, 8-*hect*, and 10M-*hect* modifiers and was visualized with anti-LexA antibody as above. Fig. 3*d* shows that, as compared with the "native" LexA-Cdk2 target, the additional lysines on the experimental target improved its ubiquitination, both increasing the amount of higher molecular weight LexA-Cdk2 derivatives, and causing the appearance of at least one new still-higher molecular weight conjugate. However, as judged by its steady-state level, the increased ubiquitination of this LexA-Lys7-Cdk2 target did not destabilize it (Fig. 3*d*).

### Example 3 : Targeted intracellular protein transporters

The possibility of using derivatized aptamers to change the localization of their protein targets *in vivo* was investigated. In the original library vector, aptamers are expressed in yeast fused to an SV40 nuclear localization sequence (NLS). LexA-fusion proteins that lack nuclear localization signals are uniformly distributed within the yeast cell (26, this study). It was tested whether anti-Cdk2 aptamers addressed to the nucleus would also concentrate LexA-Cdk2 in the nucleus. As shown by immunofluorescence experiments, LexA-Cdk2 is uniformly distributed in cells in which the chimeras B112-NLS-TrxA and B112-NLS-aptamer are not expressed. Similarly, LexA-Cdk2 is evenly distributed inside cells in which the control chimera B112-NLS-TrxA is expressed. However, in cells in which B112-NLS-anti-Cdk2 aptamers are expressed, LexA-Cdk2 is concentrated in the nucleus (Fig. 4a). All the tested fusions triggered substantial nuclear localization of their LexA-Cdk2 target.

To confirm that the concentration of LexA-Cdk2 in the nucleus was due to the nuclear translocation of peptide aptamers rather than any other aspect of the binding of aptamers to their target, LexA-Cdk2 was expressed together with either aptamers or NLS-aptamers and determined the percentage of yeast in which LexA-Cdk2 was clearly nuclear. The results show that the nuclear localization of LexA-Cdk2 depends on the expression of peptide aptamers that contain nuclear localization sequences (Fig. 4*b*). Finally, NLS-containing aptamers made against another protein, Ste5 (7) were used to determine whether NLS-aptamer fusions could cause nuclear localization of a protein that is thought to be to be predominantly cytoplasmic (27). When no aptamer was expressed or when a non-nuclear localized aptamer was expressed, LexA-Ste5 showed a predominant cytoplasmic localization. However, when NLS-aptamers were expressed, LexA-Ste5 showed a distinct concentration in the nucleus (Fig. 4*c,d*)

### Example 4 : Generation and identification of higher affinity anti-Bax aptamers :

An experiment similar to those described above for anti-CdK2 aptamers was carried out using aptamer « B16 », a Bax-interacting peptide aptamer that also recognises Nr13tr, a protein belonging to the same functional family.

The structure of the B16 aptamer was the same as those described earlier (ref. 1). Aptamer B16 carries an NLS and an Act moiety for transcriptional activation of a reporter gene in the two hybrid interaction assay.

Mutagenesis of the variable domain was carried out using mutagenic PCR as described above. Ten B16 mutants were selected for further studies.

The results of the affinity and specificity matrices are shown in Figures 5, 6 and 7. All three figures show interaction matrices, where yeast strains expressing bait proteins and conatining Lac Z reporter genes are streaked horizontally and yeast strains expressing aptamers are streaked vertically.

In contrast to the CdK2 experiments, here, aptamers having a stronger affinity to Bax, and an unchanged specificity, or even a sharper affinity towards their target, could be generated.

### Materials and methods

This section details the materials and methods used in the preceding examples.

### Identification of a higher affinity variant

The V region of anti-Cdk2 aptamer 10 was amplified from the original library vector (1) following a mutagenic PCR protocol as described (11). The purified amplified products were ligated into the RsrII-cut library vector, pJM-1 (1), which directs their conditional expression under the control of the Gall promoter, and introduced the ligation mix into *E.coli* DH5α. Plasmid DNA was prepared from a pool of 15,000 independent colonies. This pool was transformed (12) into EGY48 that contained LexA-Cdk2 (1) and pRB1840 (13) to obtain 40,000 transformants on Ura⁻His⁻Trp⁻ glucose plates. These transformants were replica plated onto Ura⁻His⁻Trp⁻ glucose /Xgal and Ura⁻His⁻Trp⁻ galactose/Xgal plates. After two days at 30°C, the 16 colonies that showed a blue color were transferred onto Ura⁻His⁻Trp⁻ glucose plates. These master plates were replica plated onto Ura⁻His⁻Trp⁻ glucose/Xgal and Ura⁻His⁻Trp⁻ galactose/Xgal plates, and confirmed that 12 of the initial 16 colonies again displayed galactose-dependent blue color. The aptamer-encoding plasmids were rescued from these strains (14) and the plasmids were reintroduced into EGY48. 7 out of these 12 plasmids conferred an interaction phenotype on galactose- but not on glucose-containing medium.

### Construction of fusion proteins

*Modifiers.* DNA encoding the *hect* domain of yeast RSP5 by PCR was isolated using the oligonucleotides 5'-ATATCTCGAGATTAAAGTACGTAGAAAGAAC-3' and 5'-ATATGTCGACGGATCCTCATTCTTGACCAAACCCTATG-3' which respectively contained an XhoI site, and BamHI and SalI sites. The PCR product was subcloned into XhoI-cut pJG4-4 (ref. 42), which contains a Trp1 marker, a 2µ replication origin, and that directed the expression of native proteins under the control of the GAL1 promoter, to create pJG4-*4(hect).* TrxA and peptide aptamers were amplified using the oligonucleotides : 5'-GGAGGCGAATTCGCCGCCACCCATGGCCGATAAAATTATTCACCTGACTGACG-3' and 5'-ATATCTCGAGCGCCAGGTTAGCGTCGAGGAAC-3', which contained respectively an EcoRI site followed by an initiator codon in a Kozak context, and an XhoI site. The PCR products were inserted into EcoRI/XhoI-cut pJG4-4 *(hect).* To express the *hect* domain only, the 5' oligonucleotide : 5'-ATATGAATTCGCCGCCACCATGGCCATTAAAGTACGTAGAAAGAACATTTTTGAG-3', was used, which contained an EcoRI site and an initiator codon in a Kozak context with the above-described 3' oligonucleotide to PCR the *hect* domain from RSP5. This fragment was introduced into EcoRI/XhoI-cut pJG4-4. The mutant *hect* domain was constructed using the Transformer kit from Clontech, according to the manufacturer's instructions, using the mutagenic oligonucleotide : 5'-GCCAAAATCTCACACAGCTTTTAACAGAGTTG-3' to change the *hect* active site cysteine to alanine, and the selection oligonucleotide : 5'-CGCTAACCTGGCGCCTAGGATTAAAGTACGTAG-3' to change to the XhoI site on the vector to an AvrII site.

For experiments with Myc-tagged ubiquitin, the modifiers were expressed from another vector. To this end, we amplified 5-*hect,* 8-*hect*, and 10M*-hect* fusions described above using oligonucleotides : 5'-ATATGTCGACGGATCCTCATTCTTGACCAAACCCTATG-3' and

5'- GGAGGCGAATTCGCCGCCACCCATGGCCGATAAAATTATTCACCTGACTGACG-3'.

The amplified products were introduced into EcoRI/XhoI cut pBC103, a plasmid that carries a Ura3 marker, a 2µ replication origin, and the GAL1 promoter.

*Myc-ubiquitin.* Yep105 contains a Trp1 marker and a 2µ replication origin and directs the expression of a Myc-tagged synthetic yeast ubiquitin gene under the control of the copper-inducible *CUP1* promoter (15).

*LexA*-*7Lys-Cdk2*. To start, the bait plasmid encoding LexA-Cdk2 (1) was used. DNA that encoded a stretch of 7 lysines was constructed by annealing the oligonucleotides : 5'-AATTGAAGAAGAAAAAAAAGAAAAAGC-3' and 5'-AATTGCTTTTTCTTTTTTTTCTTCTTC-3' and this duplex was introduced into the EcoRI site of the bait plasmid. The ligation mixture was treated with EcoRI, it was introduced into *E. coli* XL-1 blue, and cells that bore plasmids containing the insert were identified by PCR .

*Transporters.* TrxA and anti-Cdk2 peptide aptamers were amplified as described in *"modifiers".* The PCR products were introduced into EcoRI/XhoI cut pBC103 (mentioned above) and pBC104, which also carried a Ura3 marker, a 2µ replication origin, a Gall promoter, and that directed the synthesis of NLS-aptamers respectively. pJM-C6, -C7, -N2 and -N3, the plasmids that direct the synthesis of non-NLS- and NLS-tagged anti-Ste5 aptamers, are described elsewhere (7).

### Yeast manipulations

Interaction mating assays were performed as described (16) using the EGY48 strain for the preys and the EGY42 strain for the baits and reporter plasmids (17). Plasmids p3H18-34, pJK103, and pRB1840 (carrying respectively 8, 2, and 1 LexA operators upstream of a Gal1-*lacZ* fusion gene) were used in Fig. 1*a*, the pSH18-34-derived LexAop-GFP reporter (Display Systems Biotech, Copenhagen) in Fig. 1*c* and pSH18-34, in Fig. 2.

To measure interaction phenotypes with the LexAop-GFP reporter gene, overnight liquid cultures were grown from diploid exconjugants in Ura-His-Trp- galactose liquid medium. Fluorescence was measured with a FACStar plus (Becton-Dickinson) illuminated with two argon lasers tuned to 488nm and to multiline UV. Recordings were made with a 530-/+15nm filter to measure yeast fluorescence. The FL3-2 voltage (background) was set using yeast that did not show an interaction phenotype. 30,000 cells were analyzed for each interaction, and mean fluorescence of the yeast population above background was determined using the CellQuest software package (Becton-Dickinson).

To perform the modifier assays the EGY48 yeast strain was transformed with different combinations of targets and aptamer-*hect* fusions (12). Transformants were plated onto His⁻ Trp⁻ glucose plates, then colonies were grown overnight in 4ml of His⁻Trp⁻ galactose medium. For experiments with Myc-ubiquitin, EGY48 was transformed with aptamer-hect fusions and Yep105, and EGY42 was transformed with LexA-Cdk2 and pSH18-34. The strains obtained were mated and diploid exconjugants were selected on Ura-His-Trp-Leu- glucose medium. Liquid cultures were inoculated into Ura-His-Trp-Leu- galactose liquid medium, in which 100µM CuSO4 was added or not.

For western analysis (below), equal amounts of yeast were pelleted in logarithmic growth phase and the pelleted yeast was treated with zymolase (Seikagaku Ltd.) at 1mg/ml in 50□ l of (1M sorbitol, 0.5M Sodium citrate, 0.5m EDTA, 1M DTT, 1M Potassium phosphate, 0.1M PMSF) for 1hr at 30°C and lysed with SDS PAGE sample buffer.

### Immunoassays

Western blots were performed following SDS-PAGE as described (18) with rabbit anti-LexA serum (19) or rabbit anti-TrxA serum (20) secondary antibodies coupled to alkaline phosphatase, and NBT and BCIP as substrates. The blots were scanned with an optical scanner. For the Myc-Ubiquitin experiments, an ECF substrate (Amersham Pharmacia Biotech) was used and the blot was scanned with a phosphoimager (Molecular Dynamics). For immunofluorescence, aptamer expression was induced by growth in galactose for 3 hr and the cells were fixed by adding formaldehyde (3.7% final concentration) to the medium for 90 min. Samples were probed with a polyclonal rabbit anti-LexA antibody (UBI, New York), followed by affinity purified Texas Red- or fluorescein-conjugated secondary antibodies (Jackson ImmunoResearch Laboratories, PA), essentially as described (20).

### References

1. Colas, P., Cohen, B., Jessen, T., Grishina, I., McCoy, J. & Brent, R. (1996) *Nature* **380,** 548-550.
2. Xu, C. W., Mendelsohn, A. & Brent, R. (1997) *Proc. Natl. Acad. Sci. USA* **94,** 12473-12478.
3. Cohen, B. A., Colas, P. & Brent, R. (1998) *Proc. Natl. Acad. Sci. USA* **95,** 14272-14277.
4. Fabrizzio, E., Le Cam, J., Polanowska, J., Kakzorek, M., Lamb, N., Brent, R. & Sardet, C. (1999) *Oncogene* **18,** 4357-4363.
5. Butz, K., Denk, C., Ullmann, A., Scheffner, M., & Hoppe-Seyler, F. *Proc. Natl. Acad. Sci. USA* **97**, 6693-6697
6. Kolonin, M. G. & Finley, R. L., Jr. (1998) *Proc. Natl.*
   *Acad. Sci. USA* **95,** 14266-14271.
7. Geyer, C.R., Colman-Lerner, A. & Brent, R. (1999) *Proc. Natl. Acad. Sci. USA* **96,** 8567-8572.
8. Norman, T.C., Smith, D.L., Sorger, P.K., Drees, B.L., O'Rourke, S.M., Hughes, T.R., Roberts, C.J., Friend, S.H., Fields, S. & Murray, A.W. (1999) *Science* **285,** 591-595.
9. Blum, J.H., Dove, S.L., Hochschild, A., Mekalanos, J.J. (2000) *Proc. Natl. Acad. Sci USA* **97,** 2241-2246.
10. Colas, P. (2000) *Curr. Opin. Chem. Biol. 4,* 54-59.
11. Cadwell, R. C. & Joyce, G. F. (1992) *PCR Methods Appl.* **2,** 28-33.
12. Gietz, D., St. Jean, A., Woods, R. A. & Schiestl, R. H. (1992) *Nuc. Acids Res.* **20,** 1425.
13. Estojak, J., Brent, R. & Golemis, E. A. (1995) *Mol. Cell. Biol.* **15,** 5820-5829.
14. Finley, R. L., Jr. & Brent, R. (1995) in *DNA Cloning, Expression Systems: A Practical Approach,* eds. Hames, B. D. & Glover, D. M. (Oxford University Press, Oxford), pp.169-203.
15. Ellison, M.J. & Hochstrasser, M. (1991) *J. Biol. Chem.* **266,** 21150-21157.
16. Finley, R. L., Jr. & Brent, R. (1994) *Proc. Natl. Acad. Sci. USA* **91,** 2980-12984.
17. Golemis, E. A. & Brent, R. (1992) *Mol. Cell. Biol.* **12,** 3006-3014.
18. Ausubel, F. M., Brent, R., Kingston, R. E., Moore, D., Seidman, J. G. & Struhl, K. (1987-1997) (Greene and Wiley-interscience, New York).
19. Brent, R. & Ptashne, M. (1984) *Nature* **312**, 612-615.
20. LaVallie, E. R., DiBlasio, E. A., Kovacic, S., Grant, K. L., Schendel, P. F. & McCoy, J. M. (1993) *Biotechnology (N Y)* **11,** 187-193.
21. Ferrigno, P. & Silver, P. A. (1999) *Methods Cell. Biol.* **58,** 107-122.
22. Scheffner, M., Nuber, U. & Huibregtse, J. M. (1995) *Nature* **373**, 81-83.
23. Huibregtse, J. M., Scheffner, M., Beaudenon, S. & Howley, P. M. (1995) *Proc. Natl. Acad. Sci. USA* **92,** 2563-2567.
24. Jennissen, H. P. (1995) *Eur. J. Biochem.* **231,** 1-30.
25. Katti, S. K., LeMaster, D. M. & Eklund, H. (1990) *J. Mol. Biol.* **212,** 167-184.
26. Silver, P. A., Brent, R. & Ptashne, M. (1986) *Mol. Cell. Biol. **6**,* 4763-4766.
27. Mahanty, S.K., Wang, Y., Farley, F.W., Elion, E.A. (1999) *Cell* **98,** 501-512.
28. Brent, R. & Ptashne, M. (1985) *Cell* **43,** 729-736.
29. Oertel-Buchheit, P., Lamerichs, R.M., Schnarr, M. & Granger-Schnarr, M. (1990) *Mol. Gen. Genet.* **1**, 40-48.
30. Wang, G., Yang, J. & Huibregtse, J. M. (1999) *Mol. Cell. Biol.* **19,** 342-352.
31. Gosink, M. M. & Vierstra, R. D. (1995) *Proc. Natl. Acad. Sci. USA* **92,** 9117-9121.
32. Hershko, A. (1997) *Curr. Opin. Cell. Biol. **9**,* 788-799.
33. Mahaffey, D. T., Yoo, Y. & Rechsteiner, M. (1995) *FEBS Lett.* **370,** 109-112.
34. Terrell, J., Shih, S., Dunn, R. & Hicke, L. (1998) *Mol. Cell* **1,** 193-202.
35. Pelham, H. R. (1996) *Cell. Struct. Funct.* **21,** 413-419.
36. Schneider, S., Buchert, M., Georgiev, O., Catimel, B., Halford, M., Stacker, S.A., Baechi, T., Moelling, K. & Hovens, C.M. (1999) *Nature Biotech.* **17,** 170-175.
37. Haucke, V. & Lithgow, T. (1997) *J. Bioenerg. Biomembr.* **29**,11-17.
38. Boutin, J. A. (1997) *Cell Signal. **9**,* 15-35.
39. Vita, C. (1997) *Curr. Opin. Biotechnol.* **8,** 429-434.
40. Gilbert, W., de Souza, S. J. & Long, M. (1997) *Proc. Natl. Acad. Sci. USA* **94,** 7698-7703.
41. Beerli, R.R., Wels, W., Hynes, N.E., (1994), J. Biol. Chem., 269, 39, 23931-23936
42. Gyuris, J., Golemis, E., Chertkov, H & Brent, R., (1993), Cell 75, 791-803
43. Eklund et al., EMBO J. 3 : 1443-1449 (1984)
44. Holmgren, J., Biol. Chem., 264 : 13963-13966 (1989)

## Claims

1. Process for specifically modulating the properties of an intracellular target molecule T, and / or of a cellular component C which interacts directly or indirectly in a cell with T, said process comprising :
introducing into a cell a chimeric molecule, a so-called « targeted effector », comprising
- a recognition moiety R having the capacity to specifically interact, within the cell, with a site on an intracellular target molecule T, R interacting with T with a first affinity A₁ and
- an effector moiety, E covalently linked to said recognition moiety R, E being a molecule, or a portion thereof, which has an initial capacity to exert an effect on at least one molecule M, and which when it is covalently linked to R, acquires the capacity to specifically exert the effect on the intracellular target molecule T,
wherein the targeted effector interacts with T with a second affinity A₂, the affinity A₁ or the affinity A₂ corresponding to a K_{d} of less than 1 x 10⁻⁸ M, and the properties of T and / or of C are specifically modulated by the effector moiety E.

2. Process according to claim 1 wherein the binding affinity A₁ corresponds to a K_{d} of less than or equal to 1 x 10⁻⁹ M.

3. Process according to Claim 2 wherein the binding affinity A₁ corresponds to a K_{d} comprised between 1 x 10⁻⁹ M and 1 x 10⁻¹² M.

4. Process according to claim 1 wherein R is a mutant of a parent recognition moiety Rₐ, said parent recognition moiety Rₐ having the capacity to specifically interact with a site on said intracellular target molecule T, the interaction between Rₐ and T occurring with an affinity A₃, wherein the K_{d} corresponding to A₃ is greater than the K_{d} corresponding to A₁.

5. Process according to claim 1 wherein E has the initial capacity to exert an effect *in cis* and / or *in trans* on M, and, when it is covalently bound to R, acquires the capacity to exert the effect *in trans* on T.

6. Process according to claim 1 wherein the recognition moiety R is or comprises a protein or a peptide.

7. Process according to claim 6 wherein the recognition moiety R comprises a randomly generated peptide, or a part thereof.

8. Process according to claim 6 wherein the recognition moiety R comprises the binding region of a protein, or a modified version thereof.

9. Process according to any one of claims 1 to 8 wherein the recognition moiety R contains a conformationally constrained variable region displayed from a platform.

10. Process according to claim 9 wherein said platform is thioredoxin (TRX) or a TRX-like protein and said constrained variable region is a peptide having from approximately 5 to approximately 60 amino acids, preferably 10 to 40 amino acids.

11. Process according to claim 1 wherein the effect exerted by the effector moiety E involves a change in the chemical, biochemical, physical and / or functional properties of T and / or C.

12. Process according to claim 11 wherein the effector moiety E comprises an enzyme, a co-factor, an addressing signal, a transcription regulatory protein, a tracer protein, a molecule having therapeutic or diagnostic properties, a second recognition moiety, a second targeted effector, a radionuclide, a chemical modifier.

13. Process according to claim 11 wherein the effector moiety E is not a transcription activator.

14. Process according to claim 6 wherein the effector moiety E is covalently bound to the amino or carboxy terminal of the recognition moiety R.

15. Process according to claim 1 wherein the identity of T and / or C is predetermined.

16. Process according to claim 1 or 15, wherein the target molecule T or the cellular component C comprises a protein, a nucleic acid, a carbohydrate, a phosphorylated molecule, a lipid, or a combination thereof.

17. Process according to claim 1 wherein the target protein T and / or the cellular component C is/are endogenous to the cell.

18. Process according to claim 1 wherein the target protein T is a protein comprising at least two functionally distinct protein domains, Tₐ and T_{b}, and wherein the recognition moiety R specifically binds to domain Tₐ and the effector moiety E exerts the effect on domain T_{b}.

19. Process according to claim 1 wherein R has the capacity to specifically interact, within the cell, with a site on a predetermined range of target molecules, including T, and the effector moiety E, when it is covalently bound to R, acquires the capacity to exert the effect *in trans* in the cell on the said predetermined range of intracellular target molecules.

20. Process according to claim 1 wherein R interacts with a site occurring within the cell exclusively on T.

21. Process according to claim 1 wherein the recognition moiety R, in the absence of the effector moiety E, is neutral with respect to the function of the target protein T.

22. Process according to claim 1 wherein the property of T and / or C which is modulated by E is a chemical, biochemical, physical and / or functional property.

23. Process according to claim 22 wherein the modulation of the chemical, biochemical, physical, or functional properties of the target protein T or the cellular component C gives rise to a phenotypical change in the cell or organism, said phenotypical change being optionally selectable or detectable.

24. Process according to claim 22 wherein the change in the chemical properties of T and / or C comprises the formation or breakage of covalent or non-covalent bonds.

25. Process according to claim 22 wherein the change in the physical properties of T and / or C comprises a change in conformation, or in sub-cellular localisation.

26. Process according to claim 22 wherein the change in the functional properties of T and / or C comprises the enhancement or reduction in the efficiency of one or more of the functions of T and / or C, or the abolition of a function, or the creation of a new function.

27. Process according to claim 22 wherein the modulation gives rise to one or more of the following effects :
- homo- or heterodimerisation of T or C ;
- inhibition of homo- or heterodimerisation of T or C ;
- destruction of T or C ;
- stabilisation of T or C ;
- labeling of T or C ;
- ability of T or C to interact with a new partner ;
- localisation of T or C to a subcellular compartment to which T or C are not otherwise transported in the cell ;
- localisation of T or C to a subcellular compartment to which T or C are otherwise transported to a lesser degree in the cell ;
- exposure of T or C at the cell membrane ;
- internalisation of T or C ;
- provoking the association of T or C with natural intracellular partners ;
- prevention of the association of T or C with natural intracellular partners ;
- cleavage of T or C ;
- secretion of T or C.

28. Process according to claim 1 wherein the cell is a eukaryotic cell.

29. Process according to claim 1 wherein the cell is a prokaryotic cell.

30. Process according to claim 1 wherein the targeted effector is introduced into the cell by expression of a DNA sequence encoding said targeted effector as a fusion protein.

31. Process according to claim 1 wherein the targeted effector is introduced into the cell in purified form using a cell permeable agent such as a protein transduction domain (P.T.D).

32. Process according to claim 1 wherein, in a given set of conditions in the cell, the effector moiety E has the capacity to exert an effect on T when it is covalently bound to the recognition moiety, but cannot exert the said effect on T or C when it is not covalently bound to the recognition moiety, in said conditions.

33. Process according to claim 32 wherein the inability of the effector moiety E to exert the effect on T or C in the cell when it is not covalently bound to the recognition moiety is due to the inherent specificity of E or T.

34. Process according to claim 33 wherein the effector moiety E is an enzyme whose inherent specificity prevents the target protein T from acting as substrate for E when E is not covalently linked to the recognition moiety.

35. Process according to claim 32 wherein the inability of the effector moiety E to exert said effect on T or C in said cell when it is not covalently bound to the recognition moiety is due to lack of co-expression of E and T and/or C.

36. Process according to claim 32 wherein T and / or E exhibit a temporal or spatial specificity which prevents the effector moiety E and the target molecule T from associating with each other when E is not covalently linked to the recognition moiety R.

37. Process according to claim 1 wherein the effector moiety E initially has the capacity to direct an intracellular molecule M *in cis* to a predetermined sub-cellular compartment, and acquires the capacity to direct the target molecule T *in trans* to the said sub-cellular compartment when E is covalently linked to R.

38. Process for the production of a targeted effector having the capacity to specifically modulate the properties of an intracellular target molecule T, and / or a cellular component C which interacts directly or indirectly in a cell with T, said process comprising the steps :
a) production of a random pool of peptides, so-called recognition moieties R,
b) screening of the random pool produced in (a) against T in a cell, in conditions suitable to allow identification of recognition moieties R capable of interacting with T,
c) optionally contacting the moieties selected in (b) with proteins other than T to determine the specificity range of each of said moieties, and to identify moietes having a desired specificity range,
d) covalent linkage of the recognition moieties R to an effector moiety E,
E being a molecule which initially has the capacity to exert a predetermined effect on at least one intracellular component M,
step (d) being carried out either prior to step (b), or after step (e),
e) verification of the affinity A₁ with which the recognition moiety R interacts with T, or of the affinity A₂ with which the targeted effector, interacts with T,
f) if both of A₁ and A₂ correspond to K_{d} values greater than 1 x 10⁻⁸ M, alteration of the binding region of the effector moiety to adjust the binding affinity of the interaction between T and the selected moiety so that the K_{d} becomes less than 1 x 10⁻⁸ M.

39. Process according to Claim 38 wherein step (b) comprises the screening of the pool of random peptides in a cell for a detectable or selectable phenotypic change occurring via an interaction with an unknown endogenous target T.

40. Process according to Claim 39 further comprising the step of determining the identity of T by screening selected recognition moieties against molecules known to have the capacity to give rise to said phenotypic change.

41. Process according to Claim 39 further comprising the step of determining the identity of T by performing a two-hybrid screen using R or the targeted effector R-E as bait.

42. Process according to Claim 38 wherein step (b) comprises the screening of the pool of random peptides in a cell against a known target molecule T, interaction with T giving rise to a detectable or selectable change.

43. Process according to Claim 38 wherein step (b) comprises detection of reporter gene transcription in a two-hybrid interaction trap system, candidate target proteins being co-expressed in the cell with the recognition moieties.

44. Process for conferring on an effector moiety E the ability to specifically modulate the properies of an intracellular protein T, or an intracellular component C which interacts directly or indirectly with T,
said process comprising the steps of
i) covalently linking the effector moiety E to a recognition moiety R,
wherein R comprises a molecule having the capacity to specifically interact, within a cell, with a site on an intracellular target molecule T, the interaction with T occurring with an affinity A₁ which corresponds to a K_{d} value of less than 1 x 10⁻⁸ M, and
E being a molecule which has an initial capacity to exert an effect on at least one molecule M, and which when it is covalently linked to R, acquires the capacity to specifically exert the effect on the intracellular target molecule T,
ii) optionally optimising the affinity of the interaction between T and R by altering the chemical composition of the binding region of R to provide an affinity in the desired range.

45. Chimeric molecule, so-called « targeted effector », said targeted effector comprising
- a recognition moiety R, having the capacity to specifically interact, within a cell, with a site on an intracellular target molecule T, the interaction with T occurring with an affinity A₁, and
- an effector moiety, E, covalently linked to R, E being a molecule which has an initial capacity to exert an effect on at least one molecule M, and which when it is covalently linked to R, acquires the capacity to specifically exert the effect on the intracellular target molecule T,
wherein the targeted effector interacts with T with an affinity A₂, the affinity A₁ or the affinity A₂ corresponding to a K_{d} value of less than 1 x 10⁻⁸ M.

46. Chimeric molecule according to claim 45 wherein the binding affinity A₁ corresponds to a K_{d} value of less than 1 X 10⁻⁹ M.

47. Chimeric molecule according to claim 45 wherein the recognition moiety R comprises a protein or a peptide.

48. Chimeric molecule according to claim 45 wherein the recognition moiety R comprises a randomly generated peptide, or a part thereof.

49. Chimeric molecule according to claim 45 wherein the recognition moiety R comprises the binding region of a protein, or a modified version thereof.

50. Chimeric molecule according to claim 45 wherein the recognition moiety R contains a conformationally constrained variable region displayed from a platform.

51. Chimeric molecule according to claim 50 wherein said platform is thioredoxin (TRX) or a TRX-like protein and said constrained variable region is a peptide having from approximately 5 to approximately 60 amino acids, preferably 10 to 40 amino acids.

52. Chimeric molecule according to any one of claims 45 to 51 wherein the effector moiety E comprises an enzyme, a co-factor, an addressing signal, a transcription regulatory protein, a tracer protein, a target for intracellular enzymes, a molecule having therapeutic or diagnostic properties, a second recognition moiety, a second targeted effector, a radionuclide, a chemical modifier.

53. Nucleic acid encoding a chimeric protein according to claim 47 operably linked to regulatory sequences for expression in a eukaryotic cell.

54. Vector capable of stably introducing a nucleic acid according to claim 53 into a prokaryotic or eukaryotic cell.

55. Cell containing a chimeric molecule according to claim 45, or a nucleic acid according to claim 53.

56. Cell according to claim 55 which is a eukaryotic cell.

57. Cell according to claim 55 which is a prokaryotic cell.

58. Pharmaceutical composition comprising a chimeric molecule according to claim 45, or a nucleic acid according to claim 53, in association with a pharmaceutically acceptable excipient.

59. Chimeric molecule according to claim 45 for use in therapy.

60. Nucleic acid according to claim 53 for use in therapy.

61. Use of a chimeric protein according to claim 45 or a nucleic acid according to claim 53 for the preparation of a medicament for the treatment of :
- microbial infections, including viral infections, and fungal infections ;
- immunological disorders ;
- neurological disorders ;
- metabolic disorders ;
- psychiatric disorders ;
- myopathies ;
- genetic disorders ;
- cancer ;
- cardiovascular disorders ;
- dental disorders.

62. Use of a chimeric protein according to Claims 45 to 52, or a nucleic acid according to claim 53 for specifically modulating the properties of an intracellular target molecule T, and / or a cellular component C which interacts directly or indirectly in a cell with T.

63. Intracellular recognition molecule R, composed of a conformationally constrained recognition domain, displayed in a platform,
said recognition molecule having the capacity to specifically interact, within a cell, with a site on a predetermined intracellular target molecule T, the interaction with T occurring with an affinity corresponding to a K_{d} value of less than or equal to 5 x 10⁻⁹ M.

64. Intracellular recognition molecule according to claim 63, wherein the affinity of the interaction with T corresponds to a K_{d} value comprised between 1 x 10⁻⁹ M and 1 x 10⁻¹⁴ M.

65. Intracellular recognition molecule R according to claim 64 comprising a protein or a peptide.

66. Intracellular recognition molecule R according to claim 64 comprising a conformationally constrained variable region displayed from a platform

67. Intracellular recognition molecule R according to claim 66 wherein said platform is thioredoxin (TRX) or a TRX-like protein and said constrained variable region is a peptide having from approximately 5 to approximately 60 amino acids, preferably 10 to 40 amino acids.
